# NEUE EUROPÄISCHE PATENTSCHRIFT

(11) **EP 2 091 583 B2**
(45) Veröffentlichungstag und Bekanntmachung des Hinweises auf die Entscheidung über den Einspruch: **01.04.2020**
(45) Hinweis auf die Patenterteilung: 15.03.2017
(21) Anmeldenummer: 07856825.0
(22) Anmeldetag: 18.12.2007
(51) Int. Cl.: C08F 220/06, A61L 15/60, B32B 27/00, C08F 2/10, C08F 20/06, C08J 3/00, C08J 3/075, C08J 3/12, C08J 3/24, C08L 33/02, C08L 101/14, C08F 220/40, C08F 222/10, C08L 31/04, C08L 33/08, C08L 35/06

(54) **WASSERABSORBIERENDE POLYMERGEBILDE, WELCHE UNTER EINSATZ VON POLYMERDISPERSIONEN HERGESTELLT WURDEN**
WATER-ABSORBING POLYMER STRUCTURES PRODUCED USING POLYMER DISPERSIONS
STRUCTURES POLYMÈRES ABSORBANT L'EAU FABRIQUÉES EN UTILISANT DES DISPERSIONS POLYMÈRES

(30) Priorität: 18.12.2006 DE 102006060156
(43) Veröffentlichungstag der Anmeldung: 26.08.2009
(73) Patentinhaber: Evonik Operations GmbH, 45128 Essen (DE)
(72) Erfinder: LOEKER, Frank, 47798 Krefeld (DE); BREMUS, Heinz, 47809 Krefeld (DE); GERLACH, Rüdiger, 45479 Mülheim (DE); SMITH, Scott, J., Greensboro, NC 27407 (US)
(74) Vertreter: Evonik Patent Association
(86) Internationale Anmeldenummer: PCT/EP2007/011093
(87) Internationale Veröffentlichungsnummer: WO 2008/074456

(56) Entgegenhaltungen:
- EP-A1- 0 228 638
- EP-A1- 0 640 330
- EP-A2- 0 349 240
- WO-A-01/74913
- WO-A-2005/011860
- WO-A-2007/070262
- WO-A-2007/070776
- WO-A1-95/22356
- WO-A1-2010/057912
- WO-A2-2006/113561
- US-A- 4 618 631
- US-A1- 2005 096 435
- US-A1- 2005 256 757
- US-B1- 6 387 495

## Beschreibung

Die vorliegende Erfindung betrifft ein Verfahren zur Herstellung wasserabsorbierender Polymergebilde. Die Erfindung betrifft auch die durch dieses Verfahren erhältlichen wasserabsorbierenden Polymergebilde, wasserabsorbierende Polymergebilde, einen Verbund, ein Verfahren zur Herstellung eines Verbundes, den durch dieses Verfahren erhältlichen Verbund, chemische Produkte wie Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, die Verwendung wasserabsorbierender Polymergebilde sowie die Verwendung thermoplastischer Polymere.

Superabsorber sind wasserunlösliche, vernetzte Polymere, die in der Lage sind, unter Quellung und Ausbildung von Hydrogelen große Mengen an Wasser, wässrigen Flüssigkeiten, insbesondere Körperflüssigkeiten, vorzugsweise Urin oder Blut, aufzunehmen und unter Druck zurückzuhalten. Superabsorber absorbieren vorzugsweise mindestens das 100-fache ihres Eigengewichts an Wasser. Weitere Einzelheiten zu Superabsorbern sind in "Modern Superabsorbent Polymer Technology", F. L. Buchholz, A. T. Graham, Wiley-VCH, 1998" offenbart. Durch diese charakteristischen Eigenschaften sind diese wasserabsorbierenden Polymere hauptsächlich in Sanitärartikeln wie beispielsweise Babywindeln, Inkontinenzprodukten oder Damenbinden eingearbeitet.

Bei den gegenwärtig kommerziell verfügbaren Superabsorbern handelt es sich im wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind. Diese sind dadurch erhältlich, dass monomere Acrylsäure bzw. deren Salze in Gegenwart geeigneter Vernetzungsmittel radikalisch polymerisiert werden. Dabei können unterschiedliche Polymerisationsverfahren zur Anwendung gelangen, wie beispielsweise die Lösungspolymerisation, die Emulsionspolymerisation oder die Suspensionspolymerisation. Letztendlich werden durch diese unterschiedlichen Verfahren wasserabsorbierende Polymere in partikulärer Form mit einem Partikeldurchmesser in einem Bereich von 150 bis 850 µm erhalten, die dann in die Sanitärartikel eingearbeitet werden.

Um die Saug- und Quellfähigkeit dieser wasserabsorbierenden Polymerteilchen zu verbessern, wurden zahlreiche Verfahren beschrieben, in denen die Oberfläche der Polymerteilchen modifiziert wird. So ist beispielsweise aus DE 40 20 780 C1 bekannt, die wasserabsorbierenden Polymerteilchen mit Alkylencarbonaten, die mit den Carboxylgruppen der Polymerteilchen reagieren können, umzusetzen. Die auf diese Weise bewirkte Oberflächennachvernetzung führt zu einer Erhöhung der Absorption der Polymere unter der Einwirkung eines Druckes.

Neben der Umsetzung der Polymerpartikel mit reaktiven Verbindungen werden im Stand der Technik auch zahlreiche Verfahren beschrieben, mit denen die Eigenschaften der wasserabsorbierenden Polymerteilchen, insbesondere deren Permeabilität, durch eine Beschichtung mit anorganischen oder organischen Feinteilchen erzielt wird.

So wird in DE 35 03 458 A1 beschrieben, dass die Absorptionskapazität, die Absorptionsgeschwindigkeit sowie die Gelfestigkeit von Superabsorberpartikeln durch das Aufbringen von inerten anorganischen Pulvermaterialien, wie beispielsweise Siliziumdioxid, in Gegenwart von Nachvernetzungsmitteln verbessert werden kann.

Zur Verminderung der Hygroskopizität und somit zur Verminderung des Verbackens der Polymerteilchen schlägt EP 0 388 120 A1 vor, die Polymerteilchen mit einem porösen Pulver aus hochreinem Siliziumdioxid zu beschichten, wobei das Pulver eine mittlere Teilchengröße von 0,1 bis 30 µm und eine spezifische Oberfläche von 500 m²/g aufweist.

WO 01/74913 betrifft ein pulverförmiges und an der Oberfläche nachvernetztes Polymerisat. Als Nachvernetzungsmittel werden dreiwertige Salze verwendet die nach der Herstellung des Polymerisates, inklusive Zerkleinern, Mahlen und Klassieren eingesetzt werden.
Auch die WO-A-2005/011860 betrifft pulverförmige, wasserabsorbierende Polymere, beinhaltend als Bestandteile Feinteilchen mit einer Teilchengröße von weniger als 200µm, einen thermoplastischen Klebstoff sowie wasserabsorbierende Polymerteilchen mit einer Teilchengröße von 200µm und mehr, wobei die Feinteilchen mit der Oberfläche des wasserabsorbierenden Polymerteilchens über den thermoplastischen Schmelzklebstoff verbunden sind. Die Zugabe des thermoplastischen Klebstoffs auf das Vorprodukt erfolgt dabei nach dem Mahlen und vor der Nachvernetzung.
Zudem offenbart die US 2005/096435 ein superabsorbierendes Polymer, welches unter anderem, dadurch gekennzeichnet ist, dass sich auf der Oberfläche des Polymers 0,01 bis 5 Gew. % eines nicht löslichen anorganischen Pulvers und 0,01 bis 5 Gew. % thermoplastischen Polymers befinden. Erhalten werden derartige superabsorbierende Polymere dadurch, dass ein thermoplastisches Polymer auf die Oberfläche des superabsorbierenden Polymers aufgebracht wird. Die Verwendung eines thermoplastischen Polymeren in der Monomerlösung ohne weitere Zusätze, insbesondere ohne Inkontaktbringen mit organischen oder anorganischen Pulver sind im Stand der Technik jedoch nicht bekannt.

All diesen Verfahren zur nachträglichen Modifizierung ist jedoch gemein, dass sie zu einer Freisetzung von Feinteilchen in Form von Staub führen, wobei diese Feinteilchen durch mechanische Belastung, wie zum Beispiel durch pneumatische Förderung und dadurch bedingten Abrieb der wasserabsorbierenden Polymerteilchen entstehen. Neben der damit verbundenen Staubbelastung besteht ein weiterer Nachteil beim Einsatz anorganischer Feinteilchen zur Oberflächenmodifizierung wasserabsorbierender Polymergebilde darin, dass insbesondere bei einem Einsatz dieser anorganischen Feinteilchen während der Oberflächennachvernetzung die Stromaufnahme des Nachvernetzungsreaktors vergleichsweise hoch sein kann.

Der vorliegenden Erfindung lag die Aufgabe zugrunde, die sich aus dem Stand der Technik ergebenden Nachteile zu überwinden.

Insbesondere lag der vorliegenden Erfindung die Aufgabe zugrunde, wasserabsorbierende Polymergebilde bereitzustellen, welche eine vergleichsweise geringe Staubbildung aufweisen und zudem, auch in hoher Konzentration, in Hygieneartikeln eingesetzt werden können, ohne dass es zu dem so genannten *"Gel-Blocking"*-Phänomen kommt. Darüber hinaus sollten die wasserabsorbierenden Polymergebilde sich möglichst leicht in einer Vorrichtung zur Herstellung von Hygieneartikeln dosieren lassen.

Der vorliegenden Erfindung lag auch die Aufgabe zugrunde, ein Verfahren zur Herstellung wasserabsorbierender Polymergebilde mit den vorstehend beschriebenen Eigenschaften herzustellen, welches auch ohne den Einsatz feinteiliger, anorganischer Partikel durchführbar ist.

Einen Beitrag zur Lösung der eingangs genannten Aufgaben leistet ein Verfahren zur Herstellung wasserabsorbierender Polymergebilde, umfassend die Verfahrensschritte:
i) Bereitstellen einer wässrigen Monomerlösung beinhaltend
   - ein polymerisierbares, monoethylenisch ungesättigtes, eine Säuregruppe-tragendes Monomer (α1) oder ein Salz davon und Acrylsäure am meisten bevorzugt ist,
   - gegebenenfalls ein mit dem Monomer (α1) polymerisierbares, monoethylenisch ungesättigtes Monomer (α2), sowie
   - gegebenenfalls einen Vernetzer (α3),
ii) radikalische Polymerisation der wässrigen Monomerlösung unter Erhalt eines Polymergels,
iii) gegebenenfalls Zerkleinern des Polymergels,
iv) Trocknen des gegebenenfalls zerkleinerten Polymergels unter Erhalt wasserabsorbierender Polymergebilde,
v) gegebenenfalls Mahlen und Absieben der wasserabsorbierenden Polymergebilde, sowie
vi) Oberflächennachvernetzung der gegebenenfalls gemahlenen und abgesiebten wasserabsorbierenden Polymergebilde,
wobei
l) der wässrigen Monomerlösung vor dem Verfahrensschritt ii) oder während
   des Verfahrensschrittes ii), vorzugsweise vor dem Verfahrensschritt ii), oder dem Polymergel nach dem Verfahrensschritt ii) und vor dem Verfahrensschritt iv) oder während des Verfahrensschrittes iv), vorzugsweise vor dem Verfahrensschritt iv),
ein thermoplastisches Polymer in Form einer Dispersion beinhaltend das thermoplastische Polymer, ein Dispersionsmittel sowie gegebenenfalls ein Dispergiermittel zugesetzt wird, und wobei die Dispersion eine bei 20°C bestimmte Brookfield-Viskosität in einem Bereich von 0,1 bis 10.000 mPaXsec aufweist, und das Dispersionsmittel Wasser ist und die Menge an thermoplastischem Polymer in der Dispersion in einem Bereich von 5 bis 95 Gew.-%, bezogen auf das Gesamtgewicht der Dispersion, liegt und wobei das wasserabsorbierende Polymergebilde, überhaupt nicht mit einem Feinstteilchen, vorzugsweise überhaupt nicht mit einem organischen oder anorganischen Pulver in Kontakt gebracht wird.

Erfindungsgemäß bevorzugte Polymergebilde sind Fasern, Schäume oder Teilchen, wobei Fasern und Teilchen bevorzugt und Teilchen besonders bevorzugt sind.

Erfindungsgemäß bevorzugte Polymerfasern sind so dimensioniert, dass sie in oder als Garne für Textilien und auch direkt in Textilien eingearbeitet werden können. Es ist erfindungsgemäß bevorzugt, dass die Polymerfasern eine Länge im Bereich von 1 bis 500 mm, bevorzugt 2 bis 500 mm und besonders bevorzugt 5 bis 100 mm und einen Durchmesser im Bereich von 1 bis 200 Denier, bevorzugt 3 bis 100 Denier und besonders bevorzugt 5 bis 60 Denier besitzen.

Erfindungsgemäß bevorzugte Polymerteilchen sind so dimensioniert, dass sie eine mittlere Teilchengröße gemäß ERT 420.2-02 im Bereich von 10 bis 3000 µm, vorzugsweise 20 bis 2000 µm und besonders bevorzugt 150 bis 850 µm aufweisen. Dabei ist es insbesondere bevorzugt, dass der Anteil der Polymerteilchen mit einer Partikelgröße in einem Bereich von 300 bis 600 µm mindestens 30 Gew.-%, besonders bevorzugt mindestens 40 Gew.-% und am meisten bevorzugt mindestens 50 Gew.-%, bezogen auf das Gesamtgewicht der nachvernetzten, wasserabsorbierenden Polymerteilchen, beträgt.

Im Verfahrensschritt i) des erfindungsgemäßen Verfahrens wird zunächst eine wässrige Monomerlösung bereitgestellt.

Die monoethylenisch ungesättigten, säuregruppen-tragenden Monomere (α1) können teilweise odervollständig, bevorzugt teilweise neutralisiert sein. Vorzugsweise sind die monoethylenisch ungesättigten, säuregruppen-tragenden Monomere zu mindestens 25 Mol-%, besonders bevorzugt zu mindestens 50 Mol-% und darüber hinaus bevorzugt zu 50-80 Mol-% neutralisiert. In diesem Zusammenhang wird auf DE 195 29 348 A1 verwiesen. Die Neutralisation kann teilweise oder ganz auch nach der Polymerisation erfolgen. Ferner kann die Neutralisation mit Alkalimetallhydroxiden, Erdalkalimetallhydroxiden, Ammoniak sowie Carbonaten und Bicarbonaten erfolgen. Daneben ist jede weitere Base denkbar, die mit der Säure ein wasserlösliches Salz bildet. Auch eine Mischneutralisation mit verschiedenen Basen ist denkbar. Bevorzugt ist die Neutralisation mit Ammoniak und Alkalimetallhydroxiden, besonders bevorzugt mit Natriumhydroxid und mit Ammoniak.

Ferner können bei einem durch das erfindungsgemäße Verfahren erhältlichen wasserabsorbierenden Polymergebilde die freien Säuregruppen überwiegen, so dass dieses Polymergebilde einen im sauren Bereich liegenden pH-Wert aufweist. Dieses saure wasserabsorbierende Polymergebilde kann durch ein Polymergebilde mit freien basischen Gruppen, vorzugsweise Amingruppen, das im Vergleich zu dem sauren Polymergebilde basisch ist, mindestens teilweise neutralisiert werden. Diese Polymergebilde werden in der Literatur als "Mixed-Bed Ion-Exchange Absorbent Polymers" (MBIEA-Polymere) bezeichnet und sind unter anderem in der WO 99/34843 A1 offenbart. In der Regel stellen MBIEA-Polymere eine Zusammensetzung dar, die zum einen basische Polymergebilde, die in der Lage sind, Anionen auszutauschen, und andererseits ein im Vergleich zu dem basischen Polymergebilde saures Polymergebilde, das in der Lage ist, Kationen auszutauschen, beinhalten. Das basische Polymergebilde weist basische Gruppen auf und wird typischerweise durch die Polymerisation von Monomeren (α1) erhalten, die basische Gruppen oder Gruppen tragen, die in basische Gruppen umgewandelt werden können. Bei diesen Monomeren handelt es sich vor allen Dingen um solche, die primäre, sekundäre oder tertiäre Amine oder die entsprechenden Phosphine oder mindestens zwei der vorstehenden funktionellen Gruppen aufweisen. Zu dieser Gruppe von Monomeren gehören insbesondere Ethylenamin, Allylamin, Diallylamin, 4-Aminobuten, Alkyloxycycline, Vinylformamid, 5-Aminopenten, Carbodiimid, Formaldacin, Melamin und dergleichen, sowie deren sekundäre oder tertiäre Aminderivate.

Bevorzugte monoethylenisch ungesättigte, säuregruppen-tragende Monomere (α1) sind vorzugsweise diejenigen Verbindungen, die in der WO 2004/037903 A2, als ethylenisch ungesättigte, säuregruppenhaltige Monomere (α1) genannt werden. Besonders bevorzugte monoethylenisch ungesättigte, säuregruppen-tragende Monomere (α1) sind Acrylsäure und Methacrylsäure, wobei Acrylsäure am meisten bevorzugt ist.

Als mit den Monomeren (α1) copolymerisierbare, monoethylenisch ungesättigte Monomere (α2) können Acrylamide, Methacrylamide oder Vinylamide eingesetzt werden.

Bevorzugte (Meth)acrylamide sind neben Acrylamid und Methacrylamid alkylsubstituierte (Meth)acrylamide oder aminoalkylsubstituierte Derivate des (Meth)acrylamids, wie N-Methylol(meth)acrylamid, N,N-Dimethylami- no-(meth)acrylamid, Dimethyl(meth)acrylamid oder Diethyl(meth)acrylamid. Mögliche Vinylamide sind beispielsweise N-Vinylamide, N-Vinylformamide, N-Vinylacetamide, N-Vinyl-N-Methylacetamide, N-Vinyl-N-methylformamide, Vinylpyrrolidon. Unter diesen Monomeren besonders bevorzugt ist Acrylamid.

Weiterhin können als mit den Monomeren (α1) copolymerisierbare, monoethylenisch ungesättigte Monomere (α2) wasserlösliche Monomere eingesetzt werden. In diesem Zusammenhang sind insbesondere Alkoxypolyalkylenoxid(meth)acrylate wie Methoxypolyethylenglykol(meth)acrylate bevorzugt.

Als monoethylenisch ungesättigte, mit den Monomeren (α1) copolymerisierbare Monomere (α2) sind weiterhin in Wasser dispergierbare Monomere denkbar. Als in Wasser dispergierbare Monomere sind Acrylsäureester und Methacrylsäureester, wie Methyl(meth)acrylat, Ethyl(meth)acrylat, Propyl(meth)acrylat oder Butyl(meth)acrylat bevorzugt.

Die monoethylenisch ungesättigten, mit (α1) copolymerisierbaren Monomere (α2) können auch Methylpolyethylenglykolallylether, Vinylacetat, Styrol und Isobutylen umfassen.

Als Vernetzer (α3) werden vorzugsweise diejenigen Verbindungen eingesetzt, die in der WO 2004/037903 A2 als Vernetzer (α3) genannt werden. Unter diesen Vernetzern sind wasserlösliche Vernetzer besonders bevorzugt. Am meisten bevorzugt sind dabei N,N'-Methylenbisacrylamid, Polyethylenglykoldi(meth)acrylate, Triallylmethylammoniumchlorid, Tetraallylammoniumchlorid sowie mit 9 Mol Ethylenoxid pro Mol Acrylsäure hergestelltes Allylnonaethylenglykolacrylat besonders bevorzugt.

Neben den Monomeren (α1) und gegebenenfalls (α2) sowie gegebenenfalls dem Vernetzer (α3) kann die Monomerlösung auch wasserlösliche Polymere (α4) beinhalten. Bevorzugte wasserlösliche Polymere (α4) umfassend teil- oder vollverseiften Polyvinylalkohol, Polyvinylpyrrolidon, Stärke oder Stärkederivate, Polyglykole oder Polyacrylsäure. Das Molekulargewicht dieser Polymere ist unkritisch, solange sie wasserlöslich sind. Bevorzugte wasserlösliche Polymere (α4) sind Stärke oder Stärkederivate oder Polyvinylalkohol. Die wasserlöslichen Polymere (α4), vorzugsweise synthetische wie Polyvinylalkohol, können nicht nur als Pfropfgrundlage für die zu polymerisierenden Monomeren dienen. Denkbar ist auch, diese wasserlöslichen Polymere erst nach der Polymerisation mit dem Polymergel oder dem bereits getrockneten, wasserabsorbierenden Polymergel zu vermischen.

Weiterhin kann die Monomerlösung auch Hilfsmittel (α5) enthalten, wobei zu diesen Hilfsmitteln insbesondere die für die Polymerisation gegebenenfalls erforderlichen Initiatoren, Komplexbildner, wie beispielsweise EDTA, aber insbesondere auch thermoplastische Polymere oder Dispersionen beinhaltend thermoplastische Polymere gehören.

Als Lösungsmittel für die Monomerlösung kommen Wasser, organische Lösungsmittel oder Gemische aus Wasser und organischen Lösungsmitteln in Betracht, wobei die Wahl des Lösungsmittels insbesondere auch von der Art und Weise der Polymerisation abhängt.

Die relative Menge an Monomeren (α1) und (α2) sowie an Vernetzern (α3) und wasserlöslichen Polymeren (α4) und Hilfsmittel (α5) in der Monomerlösung wird vorzugsweise so gewählt, dass das im Verfahrensschritt iv) nach dem Trocknen erhaltene wasserabsorbierende Polymergebilde
- zu 20-99,999 Gew.-%, bevorzugt zu 55-98,99 Gew.-% und besonders bevorzugt zu 70-98,79 Gew.-% auf den Monomeren (α1),
- zu 0-80 Gew.-%, vorzugsweise zu 0-44,99 Gew.-% und besonders bevorzugt zu 0,1-44,89 Gew.-% auf den Monomeren (α2),
- zu 0-5 Gew.-%, vorzugsweise zu 0,001-3 Gew.-% und besonders bevorzugt zu 0,01-2,5 Gew.-% auf den Vernetzern (α3),
- zu 0-30 Gew.-%, vorzugsweise zu 0-5 Gew.-% und besonders bevorzugt zu 0,1-5 Gew.-% auf den wasserlöslichen Polymeren (α4),
- zu 0-20 Gew.-%, vorzugsweise zu 0-10 Gew.-% und besonders bevorzugt zu 0,1-8 Gew.-% auf den Hilfsmitteln (α5), und
- zu 0,5-25 Gew.-%, vorzugsweise zu 1-10 Gew.-% und besonders bevorzugt zu 3-7 Gew.-% auf Wasser (α6)
basiert, wobei die Summe der Gewichtsmengen (α1) bis (α6) 100 Gew.-% beträgt.

Optimale Werte für die Konzentration insbesondere der Monomere, Vernetzer und wasserlöslichen Polymere in der Monomerlösung können durch einfache Vorversuche ermittelt oder aber auch dem Stand der Technik, insbesondere den Druckschriften US 4,286,082, DE 27 06 135 A1, US 4,076,663, DE 35 03 458 A1, DE 40 20 780 C1, DE 42 44 548 A1, DE 43 33 056 A1 und DE 44 18 818 A1 entnommen werden.

Im Verfahrensschritt ii) des erfindungsgemäßen Verfahrens wird die im Verfahrensschritt i) erhaltene wässrige Monomerlösung unter Erhalt eines Polymergels radikalisch polymerisiert, wobei grundsätzlich alle dem Fachmann bekannten Polymerisationsverfahren in Betracht kommen können. Beispielsweise sind in diesem Zusammenhang Massepolymerisation, die vorzugsweise in Knetreaktoren wie Extrudern erfolgt, Lösungspolymerisation, Spraypolymerisation, inverse Emulsionspolymerisation und inverse Suspensionspolymerisation zu nennen.

Bevorzugt wird die Lösungspolymerisation in Wasser als Lösungsmittel durchgeführt. Die Lösungspolymerisation kann kontinuierlich oder diskontinuierlich erfolgen. Aus dem Stand der Technik ist ein breites Spektrum von Variationsmöglichkeiten hinsichtlich Reaktionsverhältnisse wie Temperaturen, Art und Menge der Initiatoren als auch der Reaktionslösung zu entnehmen. Typische Verfahren sind in den folgenden Patentschriften beschrieben: US 4,286,082, DE 27 06 135 A1, US 4,076,663, DE 35 03 458 A1, DE 40 20 780 C1, DE 42 44 548 A1, DE 43 33 056 A1, DE 44 18 818 A1. Die Polymerisation wird wie allgemein üblich durch einen Initiator ausgelöst. Als Initiatoren zur Initiierung der Polymerisation können alle unter den Polymerisationsbedingungen Radikale bildende Initiatoren verwendet werden, die üblicherweise bei der Herstellung von Superabsorbern eingesetzt werden. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der oben genannten Art durch Einwirkung energiereicher Strahlung in Gegenwart von Photoinitiatoren ausgelöst werden. Polymerisationsinitiatoren können in einer Lösung erfindungsgemäßer Monomere gelöst oder dispergiert enthalten sein. Als Initiatoren kommen sämtliche dem Fachmann bekannte in Radikale zerfallende Verbindungen in Betracht. Hierunter fallen insbesondere diejenigen Initiatoren, die bereits in der WO 2004/037903 A2 als mögliche Initiatoren genannt werden.

Besonders bevorzugt wird zur Herstellung der wasserabsorbierenden Polymergebilde ein Redoxsystem bestehend aus Wasserstoffperoxid, Natriumperoxodisulfat und Ascorbinsäure eingesetzt.

Auch die inverse Suspensions- und Emulsionspolymerisation kann im erfindungsgemäßen Verfahren angewendet werden. Gemäß diesen Prozessen wird eine wässrige, teilneutralisierte Lösung der Monomeren (α1) und (α2), gegebenenfalls beinhaltend die wasserlöslichen Polymere (α4) und Hilfsmittel (α5), mit Hilfe von Schutzkolloiden und/oder Emulgatoren in einem hydrophoben, organischen Lösungsmittel dispergiert und durch Radikalinitiatoren die Polymerisation gestartet. Die Vernetzer (α3) sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren (α4) als Pfropfgrundlage über die Monomerlösung oder durch direkte Vorlage in die Ölphase. Anschließend wird das Wasser azeotrop aus dem Gemisch entfernt und das Polymerisat abfiltriert.

Weiterhin kann sowohl bei der Lösungspolymerisation als auch bei der inversen Suspensions- und Emulsionspolymerisation die Vernetzung durch Einpolymerisation des in der Monomerlösung gelösten polyfunktionellen Vernetzers (α3) und/oder durch Reaktion geeigneter Vernetzer mit funktionellen Gruppen des Polymeren während der Polymerisationsschritte erfolgen. Die Verfahren sind beispielsweise in den Veröffentlichungen US 4,340,706, DE 37 13 601 A1, DE 28 40 010 A1 und WO 96/05234 A1 beschrieben, deren entsprechende Offenbarung hiermit als Referenz eingeführt wird.

Im Verfahrensschritt iii) des erfindungsgemäßen Verfahrens wird das im Verfahrensschritt ii) erhaltene Polymergel gegebenenfalls zerkleinert, wobei dieses Zerkleinern insbesondere dann erfolgt, wenn die Polymerisation mittels einer Lösungspolymerisation durchgeführt wird. Das Zerkleinern kann durch dem Fachmann bekannte Zerkleinerungsvorrichtungen, wie etwa einem Fleischwolf, erfolgen.

Im Verfahrensschritt iv) des erfindungsgemäßen Verfahrens wird das gegebenenfalls zuvor zerkleinerte Polymergel getrocknet. Die Trocknung des Polymergels erfolgt vorzugsweise in geeigneten Trocknern oder Öfen. Beispielhaft seien Drehrohröfen, Wirbelbetttrockner, Tellertrockner, Paddeltrockner oder Infrarottrockner genannt. Weiterhin ist es erfindungsgemäß bevorzugt, dass die Trocknung des Polymergels im Verfahrensschritt iv) bis zu einem Wassergehalt von 0,5 bis 25 Gew.-%, vorzugsweise von 1 bis 10 Gew.-% erfolgt, wobei die Trocknungstemperaturen üblicherweise in einem Bereich von 100 bis 200°C liegen.

Im Verfahrensschritt v) des erfindungsgemäßen Verfahrens können die im Verfahrensschritt iv) erhaltenen, wasserabsorbierenden Polymergebilde insbesondere dann, wenn sie durch Lösungspolymerisation erhalten wurden, noch zermahlen und auf die eingangs genannte Wunschkorngröße abgesiebt werden. Das Zermahlen der getrockneten, wasserabsorbierenden Polymergebilde erfolgt vorzugsweise in geeigneten, mechanischen Zerkleinerungsvorrichtungen, wie etwa einer Kugelmühle, während das Absieben beispielsweise durch Verwendung von Sieben mit geeigneter Maschenweite erfolgen kann.

Im Verfahrensschritt vi) des erfindungsgemäßen Verfahrens werden die gegebenenfalls zermahlenen und abgesiebten wasserabsorbierenden Polymergebilde oberflächennachvernetzt. Zur Oberflächennachvernetzung werden die getrockneten und gegebenenfalls zermahlenen und abgesiebten wasserabsorbierenden Polymergebilde aus dem Verfahrensschritt iv) oder v) aber das noch nicht getrocknete, jedoch vorzugsweise bereits zerkleinerte Polymergel aus dem Verfahrensschritt ii) oder iii) mit einem vorzugsweise organischen, chemischen Oberflächennachvernetzer in Kontakt gebracht wird. Dabei wird der Nachvernetzer insbesondere dann, wenn er unter den Nachvernetzungsbedingungen nicht flüssig ist, vorzugsweise in Form eines Fluids umfassend den Nachvernetzer sowie ein Lösungsmittel mit den wasserabsorbierenden Polymergebilde bzw. dem Polymergel in Kontakt gebracht. Als Lösungsmittel werden dabei vorzugsweise Wasser, mit Wasser mischbare organische Lösungsmittel wie etwa Methanol, Ethanol, 1-Propanol, 2-Propanol oder 1-Butanol oder Mischungen aus mindestens zwei dieser Lösungsmittel eingesetzt, wobei Wasser als Lösungsmittel am meisten bevorzugt ist. Weiterhin ist es bevorzugt, dass der Nachvernetzer in dem Fluid in einer Menge in einem Bereich von 5 bis 75 Gew.-%, besonders bevorzugt 10 bis 50 Gew.-% und am meisten bevorzugt 15 bis 40 Gew.-%, bezogen auf das Gesamtgewicht des Fluids, enthalten ist.

Das in Kontakt bringen des wasserabsorbierenden Polymergebildes bzw. des gegebenenfalls zerkleinerten Polymergels mit dem Fluid beinhaltend den Nachvernetzer erfolgt im erfindungsgemäßen Verfahren vorzugsweise durch gutes Vermischen des Fluids mit dem Polymergebilde bzw. dem Polymergel.

Geeignete Mischaggregate zum Aufbringen des Fluids sind z. B. der Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymergebilde mittels rotierender Messer in schneller Frequenz gemischt wird (Schugi-Mischer).

Das Polymergebilde bzw. das Polymergel wird in dem erfindungsgemäßen Verfahren bei der Nachvernetzung vorzugsweise mit höchstens 20 Gew. -%, besonders bevorzugt mit höchstens 15 Gew.-%, darüber hinaus bevorzugt mit höchstens 10 Gew. -%, darüber hinaus noch mehr bevorzugt mit höchstens 5 Gew.-% an Lösungsmittel, vorzugsweise Wasser, in Kontakt gebracht.

Bei Polymergebilden in der Form von vorzugsweise kugelförmigen Teilchen ist es erfindungsgemäß weiterhin bevorzugt, dass das in Kontakt bringen derart erfolgt, dass lediglich der Aussenbereich, nicht jedoch der innere Bereich der teilchenförmigen Polymergebilde mit dem Fluid und somit dem Nachvernetzer in Kontakt gebracht werden.

Als Nachvernetzer, die im erfindungsgemäßen Verfahren eingesetzt werden, werden vorzugsweise Verbindungen verstanden, die mindestens zwei funktionelle Gruppen aufweisen, die mit funktionellen Gruppen eines Polymergebildes in einer Kondensationsreaktion (=Kondensationsvernetzer), in einer Additionsreaktion oder in einer Ringöffnungsreaktion reagieren können. Als Nachvernetzer sind im erfindungsgemäßen Verfahren diejenigen bevorzugt, die in WO 2004/037903 A2 als Vernetzer der Vernetzerklassen II genannt wurden.

Unter diesen Verbindungen sind als Nachvernetzer besonders bevorzugt Kondensationsvernetzer wie beispielsweise Diethylenglykol, Triethylenglykol, Polyethylenglykol, Glyzerin, Polyglyzerin, Propylenglykol, Diethanolamin, Triethanolamin, Polyoxypropylen, Oxyethylen-Oxypropylen-Blockcopolymere, Sorbitanfettsäureester, Polyoxyethylensorbitanfettsäureester, Trimethylolpropan, Pentaerytrit, Polyvinylalkohol, Sorbitol, 1,3-Dioxolan-2-on (Ethylencarbonat), 4-Methyl-1,3-dioxolan-2-on (Propylencarbonat), 4,5-Dimethyl-1,3-dioxolan-2-on, 4,4-Dimethyl-1,3-dioxolan-2-on, 4-Ethyl-1,3-dioxolan-2-on, 4-Hydroxymethyl-1, 3-dioxolan-2-on, 1,3-Dioxan-2-on, 4-Methyl-1,3-dioxan-2-on, 4,6-Dimethyl-1,3-dioxan-2-on sowie 1,3-Dioxolan-2-on.

Nachdem die Polymergebilde bzw. die Polymergele mit dem Nachvernetzer bzw. mit dem Fluid beinhaltend den Nachvernetzer in Kontakt gebracht wurden, werden sie auf eine Temperatur im Bereich von 50 bis 300°C, vorzugsweise 75 bis 275°C und besonders bevorzugt 150 bis 250°C erhitzt, so dass, vorzugsweise wodurch, der Aussenbereich der Polymergebilde im Vergleich zum Innenbereich stärker vernetzt (=Nachvernetzung) und, sofern Polymergel eingesetzt werden, diese zugleich auch getrocknet werden. Die Zeitdauer der Wärmebehandlung wird durch die Gefahr, dass das gewünschte Eigenschaftsprofil der Polymergebilde infolge von Hitzeeinwirkung zerstört wird, begrenzt.

Gemäß dem erfindungsgemäßen Verfahren wird
l) der wässrigen Monomerlösung vor dem Verfahrensschritt ii) oder während des Verfahrensschrittes ii), vorzugsweise vor dem Verfahrensschritt ii), oder dem Polymergel nach dem Verfahrensschritt ii) und vor dem Verfahrensschritt iv) vorzugsweise vor dem Verfahrensschritt iv), ein thermoplastisches Polymer zugesetzt.

Gemäß besonderen Ausgestaltungen des erfindungsgemäßen Verfahrens wird das thermoplastische Polymer
(γ1) der wässrigen Monomerlösung vor dem Verfahrensschritt ii),
(γ2) dem Polymergel vor dem Verfahrensschritt iv),
(γ4) der wässrigen Monomerlösung vor dem Verfahrensschritt ii) und dem Polymergel vor dem Verfahrensschritt iv),
zugesetzt.

Unter einem "thermoplastischen Polymer" wird erfindungsgemäß vorzugsweise ein Polymer verstanden, dass sich unter Wärmezufuhr plastisch verformen lässt. Es ist in diesem Zusammenhang erfindungsgemäß bevorzugt, dass das thermoplastische Polymer eine durch dynamische Differenzkalorimetrie (DSC) bestimmte Schmelz- bzw. Glasübergangstemperatur in einem Bereich von -100°C bis 200°C, besonders bevorzugt -50 bis 100°C und am meisten bevorzugt -45 bis 25°C aufweist.

Erfindungsgemäß bevorzugte thermoplastische Polymere sind insbesondere Polymere ausgewählt aus der Gruppe bestehend aus Poly(meth)acrylaten, (Meth)Acrylsäure-Copolymeren, beispielsweise Ethylen-(Meth)AcrylsäureCopolymer, (Meth)Acrylsäureester-Copolymeren, Maleinsäure-Copolymeren, beispielsweise Maleinsäure-Propylen-Copolymeren, Polyurethanen, Vinylacetat-Copolymeren, beispielsweise ein Ethylen-Vinylacetat-Copolymeren oderVinylacetat-Butylacrylat-Copolymeren, Styrol-Copolymeren, beispielsweise Butylacrylat-Styrol-Copolymeren und Polycarbonaten. Dabei steht der Begriff (Meth)acrylsäure für die beiden Verbindungen Methacrylsäure und Acrylsäure, wobei von diesen beiden die Acrylsäure besonders bevorzugt ist. Erfindungsgemäß bevorzugte thermoplastische Polymere sind, was die chemische Zusammensetzung der Polymere angeht, weiterhin all diejenigen thermoplastischen Polymere, die in DE-A-103 34 286 und in WO-A- 2005/044900 als thermoplastische Polymere genannt werden.

Das durch Gelpermeationschromatographie (GPC) bestimmte Zahlenmittel des Molekulargewichts (Mₙ) der thermoplastischen Polymere liegt beispielsweise zwischen etwa 1.000 und etwa 10.000.000, zwischen etwa 20.000 und etwa 1.000.000 oder zwischen etwa 50.000 und etwa 500.000 g/Mol.

Die Molekulargewichtsverteilung der genannten Polymere, wie sie ebenfalls durch Gelpermeationschromatographie (GPC) ermittelt werden kann, kann monomodal sein. Gegebenenfalls kann ein thermoplastisches Polymer auch eine bi- oder höhermodale Verteilung aufweisen.

Die vorstehend genannten, thermoplastischen Polymere können in dem erfindungsgemäßen Verfahren als reine Substanz, beispielsweise als Polymerpartikel, oder aber auch in Form einer Dispersion beinhaltend das thermoplastische Polymere, ein Dispersionsmittel sowie gegebenenfalls ein Dispergiermittel eingesetzt werden, wobei der Einsatz in Form einer Dispersion besonders bevorzugt ist. Dabei wird unter dem Begriff "Dispersion" ein Gemenge aus dem thermoplastischen Polymer und einem Dispersionsmittel verstanden, wobei sich diese beiden Komponenten nicht oder kaum ineinander lösen oder chemisch miteinander verbinden.

Das thermoplastische Polymer ist dabei als dispergierte Phase (= disperse Phase, "innere Phase" oder Nebenphase) möglichst fein in dem Dispersionsmittel (= Dispergens, kontinuierliche Phase, "äußere Phase" oder Hauptphase) verteilt. Je nachdem, ob das thermoplastische Polymer bei der gegebenen Temperatur der Dispersion fest oder flüssig ist, kann es sich bei der Dispersion um eine Suspension oder auch um eine Emulsion handeln.

Als Dispersionsmittel können dabei insbesondere Wasser, mit Wasser mischbare organische Lösungsmittel wie etwa Methanol, Ethanol, 1-Propanol oder 2-Propanol, oder Mischungen aus Wasser und mit Wasser mischbaren organischen Lösungsmitteln eingesetzt werden, wobei der Einsatz von Wasser als Dispersionsmittel besonders bevorzugt ist.

Als Dispergiermittel können alle dem Fachmann bekannten Verbindungen eingesetzt werden, welche es ermöglichen, die vorstehend genannten thermoplastischen Polymere in Wasser oder in mit Wasser mischbaren organischen Lösungsmitteln zu dispergieren. Geeignete Dispergiermittel sind beispielsweise anionische, nichtionische, kationische oder amphotere oberflächenaktive Verbindungen wie etwa Fettsäuresalze, Kokosamine, Kokosamide und ihre Salze, Salze von Schwefelsäurealkylestern, Salze der Alkylbenzolsulfonsäure, Dialkylsulfosuccinate, Alkylphosphat-Salze und Polyoxyethylenalkylsulfat-Salze, Polyoxyethylenalkylether, Polyoxyethylenalkylphenolether, ethoxylierte Alkohole, propoxylierte Alkohole, Aminoalkohole, Polyoxyethylenfettsäureester, Sorbitanfettsäureester, Polyoxysorbitanfettsäureester, Polyoxyethylenalkylamine, Fettsäureester, Oxyethylen-Oxypropylen-Blockpolymere, Salze von Alkylaminen, quaternäre Ammoniumsalze sowie Lauryldimethylaminoxide. Derartige oberflächenaktive Substanzen können einzeln oder in Kombination miteinander zur Herstellung der Dispersionen eingesetzt werden.

Die Dispersion beinhaltend das thermoplastische Polymer, das Dispersionsmittel sowie gegebenenfalls das Dispergiermittel weist eine bei 20°C bestimmte Brookfield-Viskosität in einem Bereich von 0,1 bis 10.000 mPaXsec, besonders bevorzugt in einem Bereich von 1 bis 5.000 mPaXsec und am meisten bevorzugt in einem Bereich von 5 bis 3.000 mPaXsec auf.

Weiterhin beinhaltet die Dispersion das thermoplastische Polymer in einer Menge in einem Bereich von 5 bis 95 Gew.-%, besonders bevorzugt in einem Bereich von 20 bis 80 Gew.-%, darüber hinaus bevorzugt in einem Bereich von 30 bis 70 Gew.-% und am meisten bevorzugt in einem Bereich von 40 bis 60 Gew.-%, jeweils bezogen auf das Gesamtgewicht der Dispersion.

Erfindungsgemäß geeignete Polymerdispersionen sind beispielsweise die unter der Bezeichnung Lurapret^{®} erhältlichen Dispersionen (BASF AG, Ludwigshafen), wie beispielsweise die Produkte Lurapret^{®}DPS, Lurapret^{®}DPH, Lurapret^{®}D 312, Lurapret^{®}D 456 oder Lurapret^{®}D 500, die von der Firma Alberdingk Boley GmbH in Krefeld unterden Bezeichnungen AC 31, AC 2538, AC 2511, AC 2039, AC7574, AC 75012, AC 75030 und AC 75036 erhältlichen Dispersion, die von der Firma Ruderer Klebetechnik GmbH in Zorneding unter der Bezeichnung Ruderer 2038 erhältliche Dispersion, oder die unter der Bezeichnung Airflex^{®} von der Firma Air Products, Allentown, USA erhältlichen Produkte, wie beispielsweise Airflex^{®}315.

Wenn in dem erfindungsgemäßen Verfahren das thermoplastische Polymer der wässrigen Monomerlösung gemäß der Alternative l) vor dem Verfahrensschritt ii) oder während des Verfahrensschrittes ii) zugesetzt wird, so ist es weiterhin bevorzugt, dass das thermoplastische Polymer in einer Menge in einem Bereich von 0,01 bis 10 Gew.-%, vorzugsweise in einem Bereich von 0,05 bis 5 Gew.-% und am meisten bevorzugt in einer Menge in einem Bereich von 0,1 bis 1 Gew.-%, jeweils bezogen auf das Gesamtgewicht der Monomerlösung, der Monomerlösung zugesetzt wird. Wird das thermoplastische Polymer in Form der vorstehend beschriebenen Dispersion eingesetzt, so sind die vorstehend genannten Mengenangaben auf den Feststoffgehalt der Dispersion zu beziehen.

Wenn in dem erfindungsgemäßen Verfahren das thermoplastische Polymer dem Polymergel nach dem Verfahrensschritt ii) und vor dem Verfahrensschritt iv) gemäß der Alternative II), so ist es weiterhin bevorzugt, dass das thermoplastische Polymer in einer Menge in einem Bereich von 0,001 bis 5 Gew.-%, vorzugsweise in einem Bereich von 0,01 bis 1 Gew.-% und am meisten bevorzugt in einer Menge in einem Bereich von 0,05 bis 0,5 Gew.-%, jeweils bezogen auf das Gesamtgewicht des Polymergels bzw. des wasserabsorbierenden Polymergebildes, zugesetzt wird. Auch hier sind im Falle des Einsatzes der vorstehend beschriebenen Polymerdispersion die Mengenangeben auf den Feststoffgehalt der Polymerdispersion zu beziehen.

Wird das thermoplastische Polymer oder die Dispersion beinhaltend das thermoplastische Polymer gemäß der Alternative l) der Monomerlösung zugesetzt, so kann das thermoplastische Polymer bzw. die Dispersion vor, während oder nach der Zugabe der Monomere in einfacher Weise der Reaktionsmischung zugeführt werden. Erfolgt die Zugabe des thermoplastischen Polymers oder der Dispersion zum Polymergel vor dem Verfahrensschritt iv), so wird das thermoplastische Polymer oder die Dispersion mittels geeigneter Knetvorrichtungen, beispielsweise durch das Wölfen des Polymergels in Gegenwart des thermoplastischen Polymers oder der Dispersion in einfacher Weise in das im Verfahrensschritt ii) erhaltene Polymergel und/oder aber in das gegebenenfalls im Verfahrensschritt iii) erhaltene, zerkleinerte Polymergel eingebracht.

Weiterhin kann es vorteilhaft sein, wenn im Anschluss an den Verfahrensschritt vi) noch weitere Oberflächenmodifizierungen, wie etwa die Beschichtung der oberflächennachvernetzten wasserabsorbierenden Polymergebilde mit sogenannten "anti-cacking"-Mitteln, mit Fließhilfsmitteln wie etwa Polyethylenglykolen, mit geruchsbindenen Mitteln, wie etwa Cyclodextrinen oder Zeolithen, oder aber mit Permeabilitätssteigernden Mitteln durchgeführt werden.

Die wasserabsorbierenden Polymergebilde werden überhaupt nicht mehr mit einem Feinstteilchen, vorzugsweise nicht mehr mit einem anorganischen oder organischen Pulver, noch mehr bevorzugt nicht mehr mit einem anorganischen Pulver und am meisten besonders bevorzugt nicht mehr mit einer SiO-Verbindung, in Kontakt gebracht. Überraschenderweise wurde nämlich festgestellt, dass insbesondere durch den Einsatz von Dispersionen beinhaltend thermoplastische Polymere zur Monomerlösung oder aber auch zum wasserabsorbierenden Polymergebilde absorbierende Produkte erhalten werden können, die auch ohne einen Zusatz von Feinstteilchen, insbesondere ohne den Zusatz anorganischer Pulver, zumindest aber durch Zusatz von im Vergleich zum Stand der Technik deutlich geringeren Mengen anorganischer Pulver als permeabilitätssteigerndes Mittel oder als Mittel zur Verbesserung der Fließfähigkeit ausgezeichnete Permeabilitäten und Fließeigenschaften aufweisen. Die Formulierung "nach dem Verfahrensschritt iv) überhaupt nicht mehr mit einem Feinstteilchen in Kontakt gebracht" bedeutet dabei, dass nach dem Trocknen keine Feinstteilchen, sei es vor, während oder nach der Oberflächennachvernetzung, mehr eingesetzt werden, und zwar weder als reines Pulver noch als Fluid, beispielsweise als Suspension, beinhaltend das Feinstteilchen und ein Lösungsmittel. Gemäß einer anderen Ausgestaltung des erfindungsgemäßen Verfahrens werden auch der Monomerlösung weniger als 5 Gew.-%, vorzugsweise weniger als 1 Gew.-%, besonders bevorzugt weniger als 0,1 Gew.-%, noch mehr bevorzugt weniger als 0,01 Gew.-%, jeweils bezogen auf das Gewicht der Monomerlösung, und am meisten bevorzugt überhaupt keine Feinstteilchen, vorzugsweise kein anorganisches oder organisches Pulver, am meisten bevorzugt kein anorganisches Pulver zugesetzt.

Unter einem "Feinstteilchen", welche vorzugsweise in den vorstehend genannten Höchstmengen oder am meisten bevorzugt überhaupt nicht eingesetzt werden, werden vorzugsweise diejenigen Feinstteilchen verstanden, die in der DE 103 34 286 als Feinstteilchen genannt werden, wobei sich vorzugsweise die chemische Zusammensetzung der Feinstteilchen von der chemischen Zusammensetzung der wasserabsorbierenden Polymergebilde unterscheidet. Die Feinstteilchen weisen vorzugsweise eine durch Siebanalyse bestimmten Teilchengröße von weniger als 200 µm, besonders bevorzugt von weniger als 100 µm und am meisten bevorzugt von weniger als 50 µm auf.

Einen Beitrag zur Lösung der eingangs genannten Aufgaben leistet auch ein wasserabsorbierendes Polymergebilde, welches erhältlich ist durch das vorstehend beschriebene, erfindungsgemäße Verfahren, wobei das wasserabsorbierende Polymergebilde einen gemäß der hierin beschriebenen Testmethode für die gesamte Partikelfraktion bestimmten Fließwert von mindestens 5 und höchstens 15 aufweist. Dabei ist es besonders bevorzugt, dass das erfindungsgemäße, wasserabsorbierende Polymergebilde zu mindestens 50 Gew.-%, vorzugsweise zu mindestens 70 Gew.-% und darüber hinaus bevorzugt zu mindestens 90 Gew.-%, jeweils bezogen auf das Gewicht der wasserabsorbierenden Polymergebilde, auf carboxylatgruppen-tragenden Monomeren besteht. Es ist erfindungsgemäß weiterhin bevorzugt, dass das erfindungsgemäße, wasserabsorbierende Polymergebilde zu mindestens 50 Gew.-%, vorzugsweise zu mindestens 70 Gew.-%, jeweils bezogen auf das Gewicht der wasserabsorbierenden Polymergebilde, auf polymerisierter Acrylsäure basiert, die vorzugsweise zu mindestens 20 Mol-%, besonders bevorzugt zu mindestens 50 Mol-% und darüber hinaus bevorzugt in einem Bereich von 60 bis 85 Mol-% neutralisiert ist.

Weiterhin ist es erfindungsgemäß bevorzugt, dass das durch das erfindungsgemäße Verfahren erhältliche, wasserabsorbierende Polymergebilde durch alle folgenden Eigenschaften gekennzeichnet ist:
(β1) einen gemäß der hierin beschriebenen Testmethode für die gesamte Partikelfraktion bestimmten Staubanteil von höchstens 2 %, vorzugsweise höchstens 1,5 %, noch mehr bevorzugt höchstens 1 %, darüber hinaus bevorzugt höchstens 0,5 %, darüber hinaus noch mehr bevorzugt höchstens 0,3 %;
(β2) einen gemäß der hierin beschriebenen Testmethode für die gesamte Partikelfraktion bestimmten Fließwert von höchstens 15, vorzugsweise höchstens 10, noch mehr bevorzugt höchstens 7,5, wobei der Fließwert vorzugsweise mindestens 5, besonders bevorzugt mindestens 5,5 beträgt;
(β3) bei einer gemäß ERT 441.2-02 bestimmten Retention von < 25 g/g eine gemäß der hierin beschriebenen Testmethode bestimmte *Gel Bed Permeability* (GBP) von mindestens 50 Darcy, vorzugsweise mindestens 100 Darcy und am meisten bevorzugt mindestens 1.50 Darcy;
(β4) bei einer gemäß ERT 441.2-02 bestimmten Retention von ≥ 25 g/g und < 27 g/g eine gemäß der hierin beschriebenen Testmethode bestimmte *Gel Bed Permeability* (GBP) von mindestens 45 Darcy, vorzugsweise mindestens 90 Darcy und am meisten bevorzugt mindestens 120 Darcy;
(β5) bei einer gemäß ERT 441.2-02 bestimmten Retention von ≥ 27 g/g und < 29 g/g eine gemäß der hierin beschriebenen Testmethode bestimmte *Gel Bed Permeability* (GBP) von mindestens 40 Darcy, vorzugsweise mindestens 70 Darcy und am meisten bevorzugt mindestens 100 Darcy;
(β6) bei einer gemäß ERT 441.2-02 bestimmten Retention von ≥ 29 g/g und < 31 g/g eine gemäß der hierin beschriebenen Testmethode bestimmte *Gel Bed Permeability* (GBP) von mindestens 30 Darcy, vorzugsweise mindestens 45 Darcy und am meisten bevorzugt mindestens 60 Darcy;
(β7) bei einer gemäß ERT 441.2-02 bestimmten Retention von ≥ 31 g/g und < 33 g/g eine gemäß der hierin beschriebenen Testmethode bestimmte *Gel Bed Permeability* (GBP) von mindestens 20 Darcy, vorzugsweise mindestens 30 Darcy und am meisten bevorzugt mindestens 40 Darcy;
(β8) bei einer gemäß ERT 441.2-02 bestimmten Retention von ≥ 33 g/g und < 35 g/g eine gemäß der hierin beschriebenen Testmethode bestimmte *Gel Bed Permeability* (GBP) von mindestens 15 Darcy, vorzugsweise mindestens 20 Darcy und am meisten bevorzugt mindestens 25 Darcy;
(β9) bei einer gemäß ERT 441.2-02 bestimmten Retention von ≥ 35 g/g eine gemäß der hierin beschriebenen Testmethode bestimmte *Gel Bed Permeability* (GBP) von mindestens 8 Darcy, vorzugsweise mindestens 10 Darcy und am meisten bevorzugt mindestens 15 Darcy.

Dabei ist es bevorzugt, dass das erfindungsgemäße wasserabsorbierende Polymergebilde die gleichen Eigenschaften aufweist wie das durch das erfindungsgemäße Verfahren erhältliche wasserabsorbierende Polymergebilde. Es ist auch erfindungsgemäß bevorzugt, dass diejenigen Werte, die im Zusammenhang mit dem erfindungsgemäßen Verfahren und den erfindungsgemäßen wasserabsorbierenden Polymergebilden als Untergrenzen von erfindungsgemäßen Merkmalen ohne Obergrenzen angegeben wurden, das 20-fache, vorzugsweise das 10-fache und besonders bevorzugt das 5-fache des am meisten bevorzugten Wertes der Untergrenze besitzen.

Gemäß einer besonderen Ausführungsform des erfindungsgemäßen, wasserabsorbierenden Polymergebildes und des durch das erfindungsgemäße Verfahren erhältlichen, wasserabsorbierenden Polymergebildes beinhaltet dieses Polymergebilde weniger als 5 Gew.-%, vorzugsweise weniger als 1 Gew.-%, besonders bevorzugt weniger als 0,1 Gew.-%, noch mehr bevorzugt weniger als 0,01 Gew.-% eines Feinstteilchens, vorzugsweise eines anorganischen oder organischen Pulvers, besonders bevorzugt eines anorganischen Pulvers und am meisten bevorzugt einer SiO-Verbindung, wobei es am meisten bevorzugt ist, dass das wasserabsorbierende Polymergebilde überhaupt kein Feinstteilchen, vorzugsweise überhaupt kein anorganisches oder organisches Pulver, besonders bevorzugt überhaupt kein anorganisches Pulver, am meisten bevorzugt überhaupt keine SiO-Verbindung beinhaltet.

Einen weiteren Beitrag zur Lösung der eingangs beschriebenen Aufgaben liefert ein Verbund, beinhaltend die erfindungsgemäßen wasserabsorbierenden Polymergebilde bzw. die durch das erfindungsgemäße Verfahren erhältlichen wasserabsorbierenden Polymergebilde und ein Substrat. Es ist dabei bevorzugt, dass die erfindungsgemäßen Polymergebilde und das Substrat miteinander fest verbunden sind. Als Substrate sind Folien aus Polymeren, wie beispielsweise aus Polyethylen, Polypropylen oder Polyamid, Metalle, Vliese, Fluff, Tissues, Gewebe, natürliche oder synthetische Fasern, oder andere Schäume bevorzugt. Weiterhin ist es erfindungsgemäß bevorzugt, dass der Verbund mindestens einen Bereich umfasst, welcher das erfindungsgemäße wasserabsorbierende Polymergebilde in einer Menge im Bereich von etwa 15 bis 100 Gew.-%, vorzugsweise etwa 30 bis 100 Gew.-%, besonders bevorzugt von etwa 50 bis 99,99 Gew.-%, ferner bevorzugt von etwa 60 bis 99,99 Gew.-% und darüber hinaus bevorzugt von etwa 70 bis 99 Gew.-%, jeweils bezogen auf das Gesamtgewicht des betreffenden Bereichs des Verbundes, beinhaltet, wobei dieser Bereich vorzugsweise eine Größe von mindestens 0,01 cm³, vorzugsweise mindestens 0,1 cm³ und am meisten bevorzugt mindestens 0,5 cm³ aufweist.

In einer besonders bevorzugten Ausführungsform des erfindungsgemäßen Verbundes handelt es sich um einen flächenförmigen Verbund, wie er in der WO 02/056812 A1 als "absorbent material" beschrieben ist.

Einen weiteren Beitrag zur Lösung der Eingangs genannten Aufgaben liefert ein Verfahren zur Herstellung eines Verbundes, wobei die erfindungsgemäßen wasserabsorbierenden Polymergebilde bzw. die durch das erfindungsgemäße Verfahren erhältlichen wasserabsorbierenden Polymergebilde und ein Substrat und gegebenenfalls ein Zusatzstoff miteinander in Kontakt gebracht werden. Als Substrate werden vorzugsweise diejenigen Substrate eingesetzt, die bereits vorstehend im Zusammenhang mit dem erfindungsgemäßen Verbund genannt wurden.

Einen Beitrag zur Lösung der Eingangs genannten Aufgaben liefert auch ein Verbund erhältlich nach dem vorstehend beschriebenen Verfahren, wobei dieser Verbund vorzugsweise die gleichen Eigenschaften aufweist wie der vorstehend beschriebene, erfindungsgemäße Verbund.

Einen weiteren Beitrag zur Lösung der Eingangs genannten Aufgaben liefern chemische Produkte beinhaltend die erfindungsgemäßen Polymergebilde oder einen erfindungsgemäßen Verbund. Bevorzugte chemische Produkte sind insbesondere Schäume, Formkörper, Fasern, Folien, Filme, Kabel, Dichtungsmaterialien, flüssigkeitsaufnehmenden Hygieneartikel, insbesondere Windeln und Damenbinden, Träger für pflanzen- oder pilzwachstumsregulierende Mittel oder Pflanzenschutzwirkstoffe, Zusätze für Baustoffe, Verpackungsmaterialien oder Bodenzusätze.

Auch die Verwendung der erfindungsgemäßen Polymergebilde oder des erfindungsgemäßen Verbundes in chemischen Produkten, vorzugsweise in den vorstehend genannten chemischen Produkten, insbesondere in Hygieneartikeln wie Windeln oder Damenbinden, sowie die Verwendung der Superabsorberpartikel als Träger für pflanzen- oder pilzwachstumsregulierende Mittel oder Pflanzenschutzwirkstoffe liefern einen Beitrag zur Lösung der eingangs genannten Aufgaben. Bei der Verwendung als Träger für pflanzen- oder pilzwachstumsregulierende Mittel oder Pflanzenschutzwirkstoffe ist es bevorzugt, dass die pflanzen- oder pilzwachstumsregulierende Mittel oder Pflanzenschutzwirkstoffe über einen durch den Träger kontrollierten Zeitraum abgegeben werden können.

Einen weiteren Beitrag zur Lösung der Eingangs genannten Aufgabe liefert die Verwendung eines thermoplastischen Polymers, vorzugsweise in Form einer Dispersion beinhaltend das thermoplastische Polymer, ein Dispersionsmittel sowie gegebenenfalls ein Dispergiermittel, wie eingangs beschrieben, als Additiv für die eingangs beschriebene Monomerlösung oder als Oberflächenmodifizierungsmittel für wasserabsorbierende Polymergebilde.

Die Erfindung wird nun anhand von Figuren, Testmethoden und nicht limitierenden Beispielen nähererläutert.

### FIGUREN

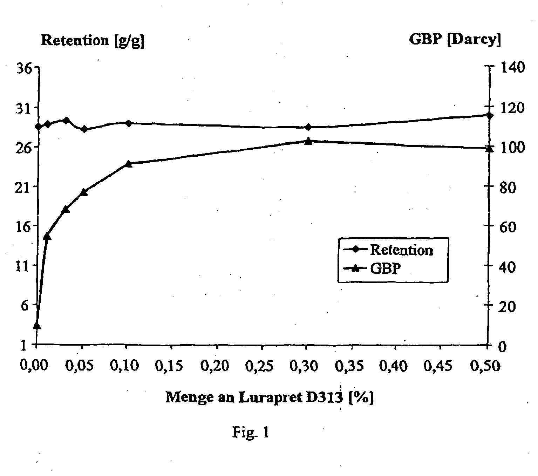

Es zeigt die Figur 1 den Einfluss steigender Mengen der thermoplastischen Polymerdispersion Lurapret^{®}D313 auf die Retention und auf die Permeabilität bei Zugabe dieser Polymerdispersion zur Nachvernetzerlösung.

### TESTMETHODEN

### Bestimmung des Fließwertes-Wertes (FFC-Wert)

Der FFC-Wert gibt Auskunft über die Fließeigenschaften eines Schüttgutes in einem Silo. Bei der Messung wird das Schüttgut verschiedenen Belastungen ausgesetzt. Das Fließverhalten lässt sich wie folgt charakterisieren;

| | |
|---|---|
| FFC < 1 | nicht fließend |
| 1 < FFC < 2 | sehr kohäsiv |
| 2 < FFC < 4 | kohäsiv |
| 4 < FFC < 10 | leicht fließend |
| 10 < FFC | frei fließend |

Gutes Fließverhalten liegt dann vor, wenn ein Schüttgut ohne großen Aufwand zum Fließen zu bringen ist, z. B. wenn das Schüttgut ohne Verfestigung aus einem Trichter oder einem Silo ausläuft. Bei schlecht fließenden Schüttgütern kommt es zu Auslaufstörungen oder sie verfestigen sich während des Transports oder der Lagerung. Mit dem Begriff "Fließen" ist gemeint, dass sich das Schüttgut aufgrund von Belastung plastisch verformt.

Weitere Angaben zu der genauen Durchführung des Tests zur Bestimmung des FFC sind den Artikeln von Herrn Dr. Ing. Dietmar Schulze "Das automatische Ringschergerät RST-01.pc" aus dem Februar 2002 und "Fließeigenschaften von Schüttgütern und verfahrenstechnische Siloauslegung" aus dem Jahr 2002 zu entnehmen. Bei dem vorliegen Messungen wurde die manuell betriebene Variante des Ringschergeräts RST-01.01 verwendet.

### Bestimmung des Staubanteils

Der Staubanteil mit einem Gerät der Firma Palas, Deutschland des Typs "Dust View" bestimmt. Hierzu wird eine Probe von 30,00 g in ein Trichterrohr gegeben. Zu Beginn der Messung öffnet sich eine Trichterklappe automatisch und die Probe fällt in ein Staubreservoir. Nun wird die Verringerung eines Laserstrahls (Abnahme der Transmission) durch die Staubbildung gemessen. Dieser Wert dient der Bestimmung des Staubanteils, d.h. der Trübung, in Prozent mit einer Skala von 1 bis 100. Der Staubanteil ergibt sich aus einem Startwert am Beginn der Messung und einem nach 30 Sekunden gemessenen Staubwert zur Bestimmung des schwebenden Anteils. Somit ergibt sich der Staubanteil aus der Summe aus Startwert und Staubwert.

### Bestimmung der Gel Bed Permeability

Die Gel Bed Permeability wird nach der in US 6,387,495 B1 offenbarten Testmethode bestimmt, wobei die Bestimmung unter Atmosphärendruck und nicht, wie in US 6,387,495 B1 beschrieben, unter Atmosphärendruck + 0,3 psi erfolgte.

### BEISPIELE

### Herstellung des wasserabsorbierenden Polymergebildes

Eine Monomerlösung bestehend aus 600 g Acrylsäure, die zu 70 Mol-% mit Natronlauge neutralisiert wurde (466,22 g 50%ige NaOH), 900,26 g Wasser, 1,44 g Polyethylenglykol-300-diacrylat, 1,44 g Monoallylpolyethylenglykol-450-monoacrylsäureester wird durch Spülen mit Stickstoff vom gelösten Sauerstoff befreit und auf die Starttemperatur von 4°C abgekühlt. Nach Erreichen der Starttemperatur wurde die Initiatorlösung (0,6 g Natriumperoxydisulfat in 10 g H₂O, 0,014 g 35%ge Wasserstoffperoxid-Lösung in 10 g H₂0 und 0,03 g Ascorbinsäure in 10 g H₂0) zugesetzt. Nachdem die Endtemperatur von ca.100°C erreicht war, wurde das entstandene Gel mit einem Fleischwolf zerkleinert und bei 150°C 2 Stunden lang im Trockenschrank getrocknet. Das getrocknete Polymerisat wurde grob zerstoßen, mittels einer Schlagkranzmühle (Firma Retsch ZM1) mit einem 5 mm-Sieb gemahlen und auf ein Pulver mit einer Partikelgröße von 150 bis 850 µm gesiebt.

### Nachvernetzung

100 g des Pulvers werden mit einer Nachvernetzungslösung bestehend aus 1,0 g Ethylencarbonat und 3 g entionisiertem Wasser mittels eines Vertikalmischer (MTI-Mischtechnik Industrieanlagen GmbH, Typ LM 1.5/5) vermischt, wobei die Lösung mittels einer Spritze mit einer 0,45 mm-Kanüle auf das sich in einem Mischer befindliche Polymerisatpulver aufgetragen wurde. Anschließend wurde das mit der wässrigen Lösung beschichtete Pulver A in einem Umluftschrank bei 185°C für 30 Minuten erhitzt. Es wird das wasserabsorbierende Polymerpulver B erhalten (Vergleichspolymer).

### Beispiel 1 (Zugabe einer Polymerdispersion zur Monomerlösung)

Das Herstellungsbeispiel wird wiederholt, wobei der Monomerlösung 1 Gew.-%, bezogen auf die Menge der Acrylsäure in der Monomerlösung, der thermoplastischen Polymeremulsion Lurapret^{®}456 (bezogen auf den Feststoffgehalt) zugesetzt werden. Es wird das wasserabsorbierende Polymerpulver C erhalten.

### Beispiel 2 (Zugabe einer Polymerdispersion zur Monomerlösung)

Das Herstellungsbeispiel wird wiederholt, wobei der Monomerlösung 1 Gew.-%, bezogen auf die Menge der Acrylsäure in der Monomerlösung, der thermoplastischen Polymeremulsion Lurapret^{®}DPS (bezogen auf den Feststoffgehalt) zugesetzt werden. Es wird das wasserabsorbierende Polymerpulver D erhalten.

### Beispiel 3 (Zugabe einer Polymerdispersion zur Monomerlösung)

Das Herstellungsbeispiel wird wiederholt, wobei der Monomerlösung 0,5 Gew.-%, 0,75 Gew.-%, 1,0 Gew.-% oder 1,5 Gew.-%, jeweils bezogen auf die Menge der Acrylsäure in der Monomerlösung, der thermoplastischen Polymeremulsion Airflex 315 zugesetzt werden. Es werden die wasserabsorbierenden Polymerpulver E, F, G und H erhalten.

### Beispiel 4 (Zugabe einer Polymerdispersion zur Monomerlösung)

Das Herstellungsbeispiel wird wiederholt, wobei der Monomerlösung 1,0 Gew.-%, bezogen auf die Menge der Acrylsäure in der Monomerlösung, der thermoplastischen Polymeremulsion ALBERDINGK^{®}AC 7502 (bezogen auf den Feststoffgehalt) zugesetzt werden. Es wird das wasserabsorbierende Polymerpulver I erhalten.

Die Eigenschaften der Polymerpulver B bis I sind in der nachfolgenden Tabelle 1 angegeben:

**Tabelle 1**

| Polymerpulver | Retention [g/g] | GBP [Darcy] |
|---|---|---|
| B | 28,4 | 14,0 |
| C | 29,0 | 90,8 |
| D | 29,0 | 94,5 |
| E | 28,6 | 74,8 |
| F | 28,7 | 79,9 |
| G | 28,3 | 107,2 |
| H | 28,4 | 113,3 |
| I | 28,0 | 126,6 |

Der Tabelle 1 ist zu entnehmen, dass die Zugabe von thermoplastischen Polymeren zur Monomerlösung zu einer signifikanten Erhöhung der Permeabilität bei etwa gleich bleibender Retention führt.

### Vergleichsbeispiel 5 (Zugabe einer Polymerdispersion während der Nachvernetzung)

Das Herstellungsbeispiel wird wiederholt, wobei der Nachvernetzerlösung die thermoplastische Polymeremulsion ALBERDINGK^{®}AC 2538 in einer solchen Menge zugesetzt wird, dass das Pulver A mit 1.000 ppm der Polymerdispersion in Kontakt gebracht wird. Es wird das wasserabsorbierende Polymerpulver J erhalten.

### Vergleichsbeispiel 6 (Zugabe einer Polymerdispersion während der Nachvernetzung)

Das Herstellungsbeispiel wird wiederholt, wobei der Nachvernetzerlösung die thermoplastische Polymeremulsion ALBERDINGK^{®}AC 75030 in einer solchen Menge zugesetzt wird, dass das Pulver A mit 1.000 ppm der Polymerdispersion in Kontakt gebracht wird. Es wird das wasserabsorbierende Polymerpulver K erhalten.

### Vergleichseispiel 7 (Zugabe einer Polymerdispersion während der Nachvernetzung)

Das Herstellungsbeispiel wird wiederholt, wobei der Nachvernetzerlösung die thermoplastische Polymeremulsion ALBERDINGK^{®}AC 75036 in einer solchen Menge zugesetzt wird, dass das Pulver A mit 1.000 ppm der Polymerdispersion in Kontakt gebracht wird. Es wird das wasserabsorbierende Polymerpulver L erhalten.

### Vergleichsbeispiel 8 (Zugabe einer Polymerdispersion während der Nachvernetzung)

Das Herstellungsbeispiel wird wiederholt, wobei der Nachvernetzerlösung die thermoplastische Polymeremulsion Lurapret^{®}D313 in einer solchen Menge zugesetzt wird, dass das Pulver A mit 3.000 ppm der Polymerdispersion (bezogen auf den Feststoffgehalt) in Kontakt gebracht wird. Es wird das wasserabsorbierende Polymerpulver M erhalten. In weiteren Versuchen wurde das thermoplastische Polymer auch in solchen Mengen zugesetzt, dass das Pulver A mit 100 ppm, 300 ppm, 500 ppm, 1.000 ppm und 5.000 ppm der Polymerdispersion in Kontakt gebracht wird. Das Ergebnis ist der Figur 1 zu entnehmen.

### Vergleichsbeispiel 9 (Zugabe einer Polymerdispersion während der Nschvernetzung)

Das Herstellungsbeispiel wird wiederholt, wobei der Nachvernetzerlösung die thermoplastische Polymeremulsion Lurapret^{®}D500 in einer solchen Menge zugesetzt wird, dass das Pulver A mit 1.000 ppm der Polymerdispersion (bezogen auf den Feststoffgehalt) in Kontakt gebracht wird. Es wird das wasserabsorbierende Polymerpulver N erhalten.

Die Eigenschaften der Polymerpulver B und J bis N sind in der nachfolgenden Tabelle 2 angegeben:

**Tabelle 2**

| Polymerpulver | Retention [g/g] | GBP [Darcy] | Staubanteil [%] |
|---|---|---|---|
| B | 28,4 | 14,0 | n. b. |
| J | 29,3 | 75,5 | 0,13 |
| K | 28,9 | 96,0 | 0,00 |
| L | 29,7 | 92,7 | 0,20 |
| M | 28,5 | 103,1 | n. b. |
| N | 28,4 | 100,5 | n. b. |

Der Tabelle 2 ist zu entnehmen, dass die Zugabe von thermoplastischen Polymeren bei der Nachvernetzung auch ohne den Zusatz anorganischer Feinstteilchen, wie etwa SiO-Pulvern, zu einer signifikanten Erhöhung der Permeabilität bei etwa gleich bleibender Retention führt. Der Figur 1 ist zu entnehmen, dass mit steigender Menge an thermoplastischem Polymer die Permeabilität steigt. Da keine Feinstteilchen zur Erzielung einer guten Permeabilität eingesetzt werden müssen, können wasserabsorbierende Pulver mit besonders geringem Staubanteil erhalten werden.

## Patentansprüche

1. Ein Verfahren zur Herstellung wasserabsorbierender Polymergebilde, umfassend die Verfahrensschritte:
i) Bereitstellen einer wässrigen Monomerlösung beinhaltend
- ein polymerisierbares, monoethylenisch ungesättigtes, eine säuregruppetragendes Monomer (α1) oder ein Salz davon,
- gegebenenfalls ein mit dem Monomer (α1) polymerisierbares, monoethylenisch ungesättigtes Monomer (α2), sowie
- gegebenenfalls einen Vernetzer (α3),
ii) radikalische Polymerisation der wässrigen Monomerlösung unter Erhalt eines Polymergels,
iii) gegebenenfalls Zerkleinern des Polymergels,
iv) Trocknen des gegebenenfalls zerkleinerten Polymergels unter Erhalt wasserabsorbierender Polymergebilde,
v) gegebenenfalls Mahlen und Absieben der wasserabsorbierenden Polymergebilde, sowie
vi) Oberflächennachvernetzung der gegebenenfalls gemahlenen und abgesiebten wasserabsorbierenden Polymergebilde,
wobei
I) der wässrigen Monomerlösung vor dem Verfahrensschritt ii) oder während des Verfahrensschrittes ii), vorzugsweise vor dem Verfahrensschritt ii), oder
II) in das Polymergel nach dem Verfahrensschritt ii) und vor dem Verfahrensschritt iv)
ein thermoplastisches Polymer in Form einer Dispersion beinhaltend das thermoplastische Polymer, ein Dispersionsmittel sowie gegebenenfalls ein Dispergiermittel zugesetzt wird, und wobei die Dispersion eine bei 20°C bestimmte Brookfield-Viskosität in einem Bereich von 0,1 bis 10.000 mPaxsec aufweist, und das Dispersionsmittel Wasser ist und die Menge an thermoplastischem Polymer in der Dispersion in einem Bereich von 5 bis 95 Gew.-%, bezogen auf das Gesamtgewicht der Dispersion, liegt und wobei das wasserabsorbierende Polymergebilde, überhaupt nicht mit einem Feinstteilchen, vorzugsweise überhaupt nicht mit einem organischen oder anorganischen Pulver in Kontakt gebracht wird.

2. Das Verfahren nach Anspruch 1, wobei das thermoplastische Polymer der wässrigen Monomerlösung vor dem Verfahrensschritt ii) zugesetzt wird.

3. Das Verfahren nach einem der vorhergehenden Ansprüche, wobei das thermoplastische Polymer eine nach ISO 11357 bestimmte Schmelz- bzw. Glasübergangstemperatur in einem Bereich von -100°C bis 200°C aufweist.

4. Das Verfahren nach einem der vorhergehenden Ansprüche, wobei das thermoplastische Polymer ein Polyacrylat, ein (Meth)AcrylsäureCopolymer, beispielsweise ein Ethylen-(Meth)Acrylsäure-Copolymer, ein (Meth)Acrylsäureester-Copolymer, ein Maleinsäure-Copolymer, beispielsweise ein Maleinsäure-Propylen-Copolymer, ein Polyurethan, ein Vinylacetat-Copolymer, beispielsweise ein Ethylen-Vinylacetat-Copolymer oder ein Vinylacetat-Butylacrylat-Copolymer, ein Styrol-Copolymer, beispielsweise ein Butylacrylat-Styrol-Copolymer, oder ein Polycarbonat ist.

5. Das Verfahren nach einem der vorhergehenden Ansprüche, wobei, wenn die Dispersion der wässrigen Monomerlösung vor dem Verfahrensschritt ii) zugesetzt wird, die Dispersion in einer Menge in einem Bereich von 0,01 bis 10 Gew.-%, bezogen auf das Gesamtgewicht der Monomerlösung, der Monomerlösung zugesetzt wird.

6. Wasserabsorbierendes Polymergebilde, erhältlich durch ein Verfahren nach einem der vorhergehenden Ansprüche, wobei das wasserabsorbierende Polymergebilde einen gemäß der hierin beschriebenen Testmethode für die gesamte Partikelfraktion bestimmten Fließwert von mindestens 5 und höchstens 15 aufweist.

7. Wasserabsorbierendes Polymergebilde nach Anspruch 6, wobei das wasserabsorbierende Polymergebilde die folgende Eigenschaftskombination aufweist:
(β1) einen gemäß der hierin beschriebenen Testmethode für die gesamte Partikelfraktion bestimmten Staubanteil von höchstens 2 %;
(β3) bei einer gemäß ERT 441.2-02 bestimmten Retention von < 25 g/g eine gemäß der hierin beschriebenen Testmethode bestimmte *Gel Bed Permeability* (GBP) von mindestens 50 Darcy;
(β4) bei einer gemäß ERT 441.2-02 bestimmten Retention von ≥ 25 g/g und < 27 g/g eine gemäß der hierin beschriebenen Testmethode bestimmte *Gel Bed Permeability* (GBP) von mindestens 45 Darcy;
(β5) bei einer gemäß ERT 441.2-02 bestimmten Retention von ≥ 27 g/g und < 29 g/g eine gemäß der hierin beschriebenen Testmethode bestimmte *Gel Bed Permeability* (GBP) von mindestens 40 Darcy;
(β6) bei einer gemäß ERT 441.2-02 bestimmten Retention von ≥ 29 g/g und < 31 g/g eine gemäß der hierin beschriebenen Testmethode bestimmte *Gel Bed Permeability* (GBP) von mindestens 30 Darcy;
(β7) bei einer gemäß ERT 441.2-02 bestimmten Retention von ≥ 31 g/g und < 33 g/g eine gemäß der hierin beschriebenen Testmethode bestimmte *Gel Bed Permeability* (GBP) von mindestens 20 Darcy;
(β8) bei einer gemäß ERT 441.2-02 bestimmten Retention von ≥ 33 g/g und < 35 g/g eine gemäß der hierin beschriebenen Testmethode bestimmte *Gel Bed Permeability* (GBP) von mindestens 15 Darcy;
(β9) bei einer gemäß ERT 441.2-02 bestimmten Retention von ≥ 35 g/g eine gemäß der hierin beschriebenen Testmethode bestimmte *Gel Bed Permeability* (GBP) von mindestens 8 Darcy.

8. Schäume, Formkörper, Fasern, Folien, Filme, Kabel, Dichtungsmaterialien, flüssigkeitsaufnehmende Hygieneartikel, Träger für pflanzen- und pilzwachstumregulierende Mittel, Verpackungsmaterialien, Bodenzusätze oder Baustoffe, beinhalten das wasserabsorbierende Polymergebilde nach einem der Ansprüche 6 bis 7.

9. Verwendung des wasserabsorbierenden Polymergebilde nach einem der Ansprüche 6 bis 7 in Schäumen, Formkörpern, Fasern, Folien, Filmen, Kabeln, Dichtungsmaterialien, flüssigkeitsaufnehmenden Hygieneartikeln, Trägern für pflanzen- und pilzwachstumregulierenden Mitteln, Verpackungsmaterialien, Bodenzusätzen, zur kontrollierten Freisetzung von Wirkstoffen oder in Baustoffen.

## Claims

1. Process for producing water-absorbing polymer structures, comprising the process steps of:
i) providing an aqueous monomer solution comprising
- a polymerizable, monoethylenically unsaturated monomer (α1) bearing acid groups, or a salt thereof,
- optionally a monoethylenically unsaturated monomer (α2) polymerizable with monomer (α1), and
- optionally a crosslinker (α3),
ii) free-radically polymerizing the aqueous monomer solution to obtain a polymer gel,
iii) optionally comminuting the polymer gel,
iv) drying the optionally comminuted polymer gel to obtain water-absorbing polymer structures,
v) optionally grinding and sieving the water-absorbing polymer structures, and
vi) surface postcrosslinking the optionally ground and sieved water-absorbing polymer structures,
by adding
I) to the aqueous monomer solution before process step ii) or during process step ii), preferably before process step ii), or
II) to the polymer gel after process step ii) and before process step iv)
a thermoplastic polymer in the form of a dispersion comprising the thermoplastic polymer, a dispersion medium and optionally a dispersant, and wherein the dispersion has a Brookfield viscosity determined at 20°C within a range from 0.1 to 10 000 mPa × sec, and the dispersion medium is water, and the amount of thermoplastic polymer in the dispersion is within a range from 5 to 95% by weight, based on the total weight of the dispersion, and wherein the water-absorbing polymer structure is not contacted at all with ultrafine particles, preferably not at all with an organic or inorganic powder.

2. Process according to Claim 1, wherein the thermoplastic polyester is added to the aqueous monomer solution before process step ii).

3. Process according to either of the preceding claims, wherein the thermoplastic polymer has a melting temperature or glass transition temperature determined to ISO 11357 within a range from -100°C to 200°C.

4. Process according to one of the preceding claims, wherein the thermoplastic polymer is a polyacrylate, a (meth)acrylic acid copolymer, for example an ethylene-(meth)acrylic acid copolymer, a (meth)acrylic ester copolymer, a maleic acid copolymer, for example a maleic acid-propylene copolymer, a polyurethane, a vinyl acetate copolymer, for example an ethylene-vinyl acetate copolymer or a vinyl acetate-butyl acrylate copolymer, a styrene copolymer, for example a butyl acrylate-styrene copolymer, or a polycarbonate.

5. Process according to one of the preceding claims, wherein, when the dispersion is added to the aqueous monomer solution before process step ii), the dispersion is added to the monomer solution in an amount within a range from 0.01 to 10% by weight, based on the total weight of the monomer solution.

6. Water-absorbing polymer structure obtainable by a process according to one of the preceding claims, wherein the water-absorbing polymer structure has a flow value determined by the test method described herein for the entire particle fraction of at least 5 and at most 15.

7. Water-absorbing polymer structure according to Claim 6, which has the following combination of properties:
(β1) a dust content determined by the test method described herein for the entire particle fraction of at most 2%;
(β3) at a retention determined to ERT 441.2-02 of < 25 g/g, a *Gel Bed Permeability* (GBP) determined by the test method described herein of at least 50 darcies;
(β4) at a retention determined to ERT 441.2-02 of ≥ 25 g/g and < 27 g/g, a *Gel Bed Permeability* (GBP) determined by the test method described herein of at least 45 darcies;
(β5) at a retention determined to ERT 441.2-02 of ≥ 27 g/g and < 29 g/g, a *Gel Bed Permeability* (GBP) determined by the test method described herein of at least 40 darcies;
(β6) at a retention determined to ERT 441.2-02 of ≥ 29 g/g and < 31 g/g, a *Gel Bed Permeability* (GBP) determined by the test method described herein of at least 30 darcies;
(β7) at a retention determined to ERT 441.2-02 of ≥ 31 g/g and < 33 g/g, a *Gel Bed Permeability* (GBP) determined by the test method described herein of at least 20 darcies;
(β8) at a retention determined to ERT 441.2-02 of ≥ 33 g/g and < 35 g/g, a *Gel Bed Permeability* (GBP) determined by the test method described herein of at least 15 darcies;
(β9) at a retention determined to ERT 441.2-02 of ≥ 35 g/g, a *Gel Bed Permeability* (GBP) determined by the test method described herein of at least 8 darcies.

8. Foam, moulding, fibre, foil, film, cable, sealing material, liquid-absorbing hygiene article, carrier for crop growth and fungal growth regulators, packaging material, soil additive or building material, comprising the water-absorbing polymer structure according to one of Claims 6 to 7.

9. Use of the water-absorbing polymer structure according to one of Claims 6 to 7 in foams, mouldings, fibres, foils, films, cables, sealing materials, liquid-absorbing hygiene articles, carriers for crop growth and fungal growth regulators, packaging materials, soil additives, for controlled release of active ingredients or in building materials.

## Revendications

1. Procédé de fabrication de structures polymères hydroabsorbantes, comprenant les étapes de procédé de :
i) préparation d'une solution aqueuse de monomère contenant :
- un monomère (α1) polymérisable, insaturé monoéthyléniquement, porteur d'un groupe acide ou un sel de celui-ci,
- éventuellement un monomère (α2) polymérisable avec le monomère (α1), insaturé monoéthyléniquement, ainsi que
- éventuellement un agent réticulant (α3),
ii) polymérisation radicalaire de la solution aqueuse de monomère en obtenant d'un gel de polymère,
iii) éventuellement découpe en petits morceaux du gel de polymère,
iv) séchage du gel de polymère éventuellement découpé en petits morceaux en obtenant des structures polymères hydroabsorbantes,
v) éventuellement broyage et tamisage des structures polymères hydroabsorbantes, ainsi que
vi) post-réticulation de surface des structures polymères hydroabsorbantes éventuellement broyées et tamisées,
où on ajoute,
I) à la solution aqueuse de monomère avant l'étape de procédé ii) ou pendant l'étape de procédé ii), préférentiellement avant l'étape de procédé ii), ou
II) dans le gel de polymère après l'étape de procédé ii) et avant l'étape de procédé iv),
un polymère thermoplastique sous forme d'une dispersion contenant le polymère thermoplastique, un agent dispersant ainsi, éventuellement, qu'un agent dispersant,
et où la dispersion présente une viscosité Brookfield déterminée à 20°C dans une plage de 0,1 à 10 000 mPa.s, et où l'agent dispersant est de l'eau, et la quantité de polymère thermoplastique dans la dispersion est dans une plage de 5 à 95 % en poids, rapporté au poids total de la dispersion, et où les structures hydroabsorbantes ne sont absolument pas mises en contact avec une particule fine, de préférence absolument pas avec une poudre organique ou minérale.

2. Procédé selon la revendication 1, où on ajoute le polymère thermoplastique à la solution aqueuse de monomère avant l'étape de procédé ii).

3. Procédé selon une des revendications précédentes, dans lequel le polymère thermoplastique présente une température de fusion ou respectivement de transition vitreuse déterminée selon ISO 11357 dans une plage de -100°C à 200°C.

4. Procédé selon une des revendications précédentes, dans lequel le polymère thermoplastique est un polyacrylate, un copolymère d'acide (méth)acrylique, par exemple un copolymère éthylène-acide (méth)acrylique, un copolymère d'ester d'acide (méth)acrylique, un copolymère d'acide maléique, par exemple un copolymère acide maléique-propylène, un polyuréthane, un copolymère d'acétate de vinyle, par exemple un copolymère éthylène-acétate de vinyle ou un copolymère acétate de vinyle-acrylate de butyle, un copolymère de styrène, par exemple un copolymère acrylate de butyle-styrène, ou un polycarbonate.

5. Procédé selon une des revendications précédentes, dans lequel, si la dispersion est ajoutée à la solution aqueuse de monomère avant l'étape de procédé ii), on l'ajoute à la solution de monomère en une quantité dans la plage de 0,01 à 10 % en poids rapporté au poids total de la solution de monomère.

6. Structure polymère hydroabsorbante qui s'obtient par un procédé selon une des revendications précédentes, où la structure polymère hydroabsorbante présente une valeur de fluage d'au moins 5 et au maximum de 15 déterminée selon la méthode de test décrite dans la présente pour la fraction totale de particules.

7. Structure polymère hydroabsorbante selon la revendication 6, la structure polymère hydroabsorbante présentant la combinaison de propriétés suivante :
(β1) une teneur en poussières de maximum 2 % déterminée selon la méthode de test décrite dans la présente pour la fraction totale de particules ;
(β3) une perméabilité de lit de gel *(Gel Bed Permeability)* (GBP) d'au moins 50 Darcy déterminée selon la méthode de test décrite dans la présente pour une rétention de < 25 g/g déterminée selon ERT 441.2-02 ;
(β4) une perméabilité de lit de gel *(Gel Bed Permeability*) (GBP) d'au moins 45 Darcy déterminée selon la méthode de test décrite dans la présente pour une rétention de ≥ 25 g/g et < 27 g/g déterminée selon ERT 441.2-02 ;
(β5) une perméabilité de lit de gel *(Gel Bed Permeability)* (GBP) d'au moins 40 Darcy déterminée selon la méthode de test décrite dans la présente pour une rétention de ≥ 27 g/g et < 29 g/g déterminée selon ERT 441.2-02 ;
(β6) une perméabilité de lit de gel *(Gel Bed Permeability*) (GBP) d'au moins 30 Darcy déterminée selon la méthode de test décrite dans la présente pour une rétention de ≥ 29 g/g et < 31 g/g déterminée selon ERT 441.2-02 ;
(β7) une perméabilité de lit de gel *(Gel Bed Permeability)* (GBP) d'au moins 20 Darcy déterminée selon la méthode de test décrite dans la présente pour une rétention de ≥ 31 g/g et < 33 g/g déterminée selon ERT 441.2-02 ;
(β8) une perméabilité de lit de gel *(Gel Bed Permeability*) (GBP) d'au moins 15 Darcy déterminée selon la méthode de test décrite dans la présente pour une rétention de ≥ 33 g/g et < 35 g/g déterminée selon ERT 441.2-02 ;
(β9) une perméabilité de lit de gel *(Gel Bed Permeability*) (GBP) d'au moins 8 Darcy déterminée selon la méthode de test décrite dans la présente pour une rétention de ≥ 35 g/g déterminée selon ERT 441.2-02.

8. Mousses, articles moulés, fibres, feuillets, films, câbles, matériaux d'étanchéité, articles d'hygiène absorbeur de liquides, vecteurs pour produits régulateurs de croissance de plantes et de champignons, matériaux d'emballage, engrais ou matériaux de construction, contenant la structure polymère hydroabsorbante selon une des revendications 6 et 7.

9. Utilisation de la structure polymère hydroabsorbante selon une des revendications 6 et 7 dans des mousses, articles moulés, fibres, feuillets, films, câbles, matériaux d'étanchéité, articles d'hygiène absorbeur de liquides, vecteurs pour produits régulateurs de croissance de plantes et de champignons, matériaux d'emballage, engrais, pour contrôler la libération de substances actives ou dans des matériaux de construction.
